# EUROPÄISCHE PATENTSCHRIFT

(11) **EP 0 280 088 B1**
(45) Veröffentlichungstag und Bekanntmachung des Hinweises auf die Patenterteilung: **26.04.1995**
(21) Anmeldenummer: 88101638.0
(22) Anmeldetag: 04.02.1988
(51) Int. Cl.: A61B 17/22

(54) **Schallerzeuger zur Behandlung eines Lebewesens mit fokussierten Schallwellen**
Sound generator for treating a living being with focused sound waves
Générateur de son pour le traitement d'un être vivant avec des ondes sonores focalisées

(30) Priorität: 16.02.1987 DE 3704836
(43) Veröffentlichungstag der Anmeldung: 31.08.1988
(73) Patentinhaber: SIEMENS AKTIENGESELLSCHAFT, 80333 München (DE)
(72) Erfinder: Wessels, Gerd, Dr., D-8520 Erlangen (DE)

(56) Entgegenhaltungen:
- EP-A- 194 897
- EP-A- 0 148 653
- DE-A- 3 328 039
- US-A- 4 315 514

## Beschreibung

Die Erfindung betrifft einen Schallerzeuger zur Behandlung eines Lebewesens mit fokussierten Schallwellen, welcher eine elektrisch antreibbare Schallquelle, Fokussierungsmittel für die von der Schallquelle ausgehenden Schallwellen und eine die Schallquelle antreibende elektrische Generatoreinrichtung aufweist.

Derartige Schallerzeuger, die in der Regel Schallwellen im Ultraschallbereich abgeben, finden z.B. bei der Behandlung von pathologischen Gewebeveränderungen, z.B. Tumoren, Verwendung. Dabei geben die Schallerzeuger für die jeweilige Behandlungsart geeignet ausgebildete Schallwellen im wesentlichen als Dauerschall ab.

Solche Schallerzeuger sind aus der US-PS 4 315 514 und der EP-A-0 194 897 bekannt. Als Generatoreinrichtung ist im Falle der US-PS 4 315 514 ein Radiofrequenz-Generator vorgesehen, der eine periodische Wechselspannung geeigneter Frequenz an die Schallquelle liefert. Es wird also Dauerschall erzeugt. Die Schallquelle gemäß der EP-A-0 194 897 ist entweder zur Behandlung von pathologischen Gewebeveränderungen mit Dauerschall oder zur berührungslosen Zerstörung von Konkrementen im Körper eines Patienten geeignet und wird jeweils zur Bilderzeugung eingesetzt.

Aus der EP-A-0 148 653 ist ein Schallerzeuger gemäß dem Oberbegriff des Patentanspruches 1 bekannt, bei dem der zweite Generator intermittierende Impulsreihen abgibt, die für Behandlungszwecke nicht vorgesehen sind.

Es hat sich gezeigt, daß bei der Behandlung pathologischer Gewebeveränderung bessere Erfolge erzielt werden, wenn die zu behandelnden Gewebezonen außer mit Dauerschall auch mit andersartig ausgebildeten Schallwellen, z.B. Stoßwellen, beaufschlagt werden, da auf diese Weise eine nachhaltige Störung des Stoffwechsels der Zellen erreicht werden kann, die deren Absterben nach sich ziehen kann.

Aus der DE-OS 33 19 871 und der bereits erwähnten EP-A-0 148 653 sind zwar bereits Schallerzeuger bekannt, die von Dauerschall abweichende Schallwellen, nämlich Stoßwellen, abgeben.

Diese Schallerzeuger sind jedoch zur berührungslosen Zerstörung von Konkrementen im Körper eines Patienten vorgesehen und zur Behandlung von pathologischen Gewebeveränderungen ungeeignet. Beide Schallerzeuger enthalten zusätzliche Mittel zur Erzeugung diagnostischer Schallwellen, die nicht der Behandlung, sondern der Ortung des zu zertrümmernden Konkrementes dienen. Dabei erfolgt die Erzeugung der diagnostischen Schallwellen zumindest im Falle der EP-A-0 148 653 intermittierend.

Selbst wenn man die zuletzt genannten Schallerzeuger den Bedürfnissen bei der Behandlung von Gewebe anpassen würde, wären zur Behandlung von pathologischen Gewebeveränderungen mit Dauerschall und Stoßwellen zwei Schallerzeuger erforderlich, da die bekannten Schallerzeuger jeweils nur eine Art von Schallwellen abgeben können. Dies ist umständlich und steht der Einführung der erwähnten Behandlungsart in der Praxis entgegen.

Der Erfindung liegt die Aufgabe zugrunde, einen Schallerzeuger der eingangs genannten Art so auszubilden, daß er unterschiedliche Arten von Schallwellen abgeben kann, die z.B. für die Behandlung von pathologischen Gewebeveränderungen geeignet sind.

Nach der Erfindung wird diese Aufgabe dadurch gelöst, daß der zweite Generator zur Erzeugung von Dauerschall ausgebildet ist. Dabei kann nach einer Variante der Erfindung vorgesehen sein, daß die Schallquelle wahlweise an einen der Generatoren anschaltbar ist. Die Schallquelle kann aber auch aus wenigstens zwei unabhängig voneinander antreibbaren Schallwandlern bestehen, von denen jeder an jeweils einen Generator anschaltbar ist. Es besteht dann die Möglichkeit, die Schallwandler an die jeweils abzustrahlende Art von Schallwellen optimal anzupassen. Außerdem besteht in diesem Fall die Möglichkeit, mehrere Schallwandler mittels der jeweiligen Generatoren gleichzeitig anzutreiben, so daß mehrere Arten von Schallwellen gleichzeitig auf die zu behandelnde Gewebezone einwirken.

Es kann vorgesehen sein, daß der Generator zur Erzeugung von Dauerschall intermittierend betreibbar ist. Es besteht dann die Möglichkeit, das Zeitintervall zwischen der Abgabe von Dauerschall dazu auszunutzen, mit Hilfe der Echos der von dem Schallerzeuger ausgesandten Schallwellen eine Ortung des zu behandelnden Gebietes im Körperinneren vorzunehmen. Auch im Falle der Abgabe von Stoßwellen besteht die Möglichkeit, das Zeitintervall zwischen zwei aufeinanderfolgenden Stoßwellen zur Ortung zu nutzen. Die Ortung ist auf besonders einfache Weise möglich, wenn nach einer Ausführungsform der Erfindung die Schallquelle durch wenigstens einen piezoelektrischen Wandler gebildet ist, da dieser nicht nur die Schallwellen aussenden, sondern deren Echos auch mit ausreichender Empfindlichkeit empfangen kann.

Im Falle der Verwendung eines piezoelektrischen Wandlers als Schallquelle ist nach einer Variante der Erfindung vorgesehen, daß der piezoelektrische Wandler zur Erzeugung von Stoßwellen an einen Hochspannungsimpulse abgebenden Generator und zur Erzeugung von Dauerschall bzw. intermittierendem Dauerschall an einen eine periodische bzw. intermittierende periodische Wechselspannung liefernden Generator anschaltbar ist.

Zwei Ausführungsbeispiele der Erfindung sind in den Fig. der beigefügten Zeichnungen schematisch dargestellt.

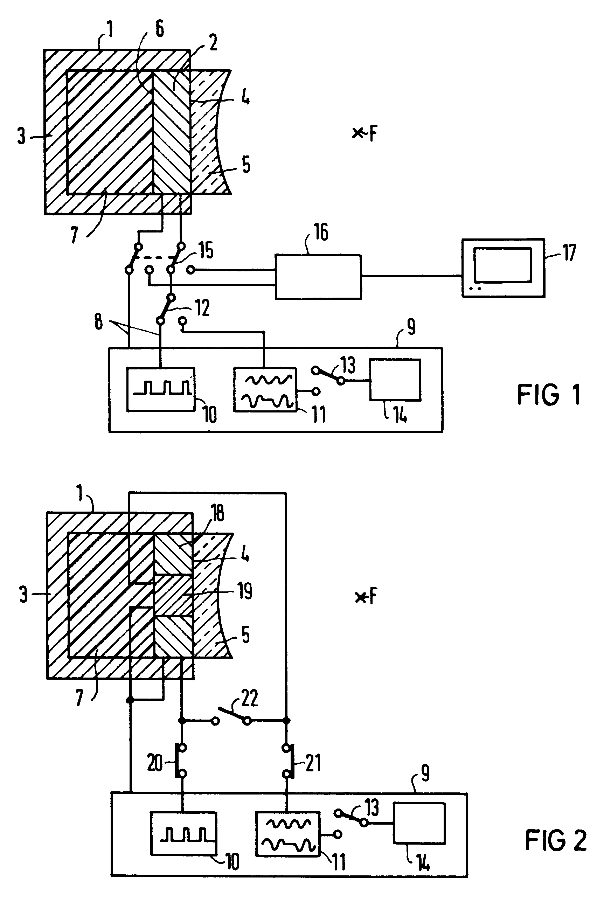

Die Fig. 1 zeigt einen erfindungsgemäßen Schallerzeuger, welcher als elektrisch antreibbare Schallquelle einen in einem topfartigen Gehäuse 1 angeordneten, scheibenförmig ausgebildeten piezoelektrischen Wandler 2 aufweist. Mit der von dem Boden 3 des Gehäuses 1 abgewandten Stirnfläche 4 des piezoelektrischen Wandlers 2 ist eine akustische Linse 5 zur Fokussierung der ausgesandten Schallwellen verbunden, während an seiner anderen Stirnfläche ein Dämpfungskörper 7 zur Bedämpfung der von der Stirnfläche 6 ausgehenden Schallwellen vorgesehen ist. Der piezoelektrische Wandler 2 ist über eine zweiadrige Leitung 8 mit einer Generatoreinrichtung 9 verbunden. Diese weist zwei Generatoren 10 und 11 auf, an die der piezoelektrische Wandler 2 mittels eines Schalters 12 wahlweise anschaltbar ist. Dabei erhält der piezoelektrische Wandler 2, wenn er an den Generator 10 angeschaltet ist, in der Fig. 1 schematisch angedeutete Hochspannungsimpulse zur Erzeugung von Stoßwellen. Wenn der piezoelektrische Wandler 2 dagegen an den Generator 11 angeschaltet ist, erhält er eine in der Fig. 1 schematisch angedeutete periodische Wechselspannung zur Erzeugung von Dauerschall.

Der Generator 10 ist über einen weiteren mit 13 bezeichneten Schalter mit einer Steuereinrichtung 14 verbunden. Wenn der Schalter 13 geschlossen ist, wird der Generator 11 durch die Steuereinrichtung 14 derart angesteuert, daß er zur Erzeugung von intermittierendem Dauerschall eine in der Fig. 1 schematisch angedeutete intermittierende periodische Wechselspannung an den piezoelektrischen Wandler 2 abgibt.

Zwischen der Generatoreinrichtung 9 und dem piezoelektrischen Wandler 2 sind Schaltmittel 15 vorgesehen, mittels derer der piezoelektrische Wandler 2 wahlweise statt an die Generatoreinrichtung 9 an eine Meßeinrichtung 16 anschaltbar ist, die mit einem Sichtgerät 17 verbunden ist. Es besteht dann die Möglichkeit, die im Bereich des Fokus F befindliche Gewebezone mittels der Echos der von dem Wandler 2 ausgesandten Schallwellen auf dem Sichtgerät 17 abzubilden. Zu diesem Zweck sind die Schaltmittel 15 in nicht dargestellter Weise mit der Steuereinrichtung bzw. mit dem Generator 10 verbunden und werden derart gesteuert, daß im Falle der Aussendung von Stoßwellen bzw. von intermittierendem Dauerschall der piezoelektrische Wandler 2 während der Abgabe einer Stoßwelle bzw. von Dauerschall mit der Generatoreinrichtung 9 und in den dazwischenliegenden Zeitintervallen mit der Meßeinrichtung 16 verbunden ist.

Der in Figur 2 dargestellte Schallerzeuger, bei dem die Einrichtung zur Ortung der zu behandelnden Gewebezone nicht dargestellt ist, entspricht im wesentlichen dem zuvor beschriebenen, weshalb gleiche Teile mit den gleichen Bezugszeichen versehen sind. Der wesentliche Unterschied zu dem zuvor beschriebenen Schallerzeuger besteht darin, daß die Schallquelle aus zwei voneinander unabhängigen piezoelektrischen Wandlern 18 und 19 besteht, wobei der Wandler 18 über den Schalter 20 an den Generator 10 und der Wandler 19 über den Schalter 21 an den Generator 11 anschaltbar ist. Im einzelnen ist der piezoelektrische Wandler 18 kreisringförmig ausgebildet und zur Abgabe von Stoßwellen vorgesehen. Der scheibenförmig ausgebildete, zur Abgabe von Dauerschall bzw. intermittierendem Dauerschall vorgesehene piezoelektrische Wandler 19 ist in der Öffnung des piezoelektrischen Wandlers 18 angeordnet. Die Schalter 20 und 21 sind unabhängig voneinander betätigbar. Es sind somit drei Betriebsarten möglich. Es handelt sich dabei um die Abgabe von Stoßwellen, mittels des piezoelektrischen Wandlers 18 und des Generators 10 bei geschlossenem Schalter 20 und geöffnetem Schalter 21, die Abgabe von Dauerschall mittels des piezoelektrischen Wandlers 19 und des Generators 11 bei geschlossenem Schalter 21 und geöffnetem Schalter 20 und die Abgabe von Dauerschall und Stoßwellen mittels der piezoelektrischen Wandler 18 und 19 und der Generatoren 10 und 11 bei geschlossenen Schaltern 20 und 21. Diese Betriebsart ist in Fig. 2 dargestellt.

Es ist außerdem ein Schalter 22 vorgesehen, der es gestattet, die beiden piezoelektrischen Wandler 18 und 19 einander parallel zu schalten. Es ist dann möglich, beide piezoelektrischen Wandler 18 und 19 gemeinsam an einem der Generatoren 10 oder 11 zu betreiben, je nach dem ob außer dem Schalter 22 der Schalter 20 geschlossen und der Schalter 21 geöffnet ist oder umgekehrt.

## Patentansprüche

1. Schallerzeuger zur Behandlung eines Lebewesens mit fokussierten Schallwellen, welcher eine elektrisch antreibbare Schallquelle (2; 18, 19), Fokussierungsmittel (5) für die von der Schallquelle (2; 18, 19) ausgehenden Schallwellen und eine die Schallquelle (2; 18, 19) antreibende elektrische Generatoreinrichtung (9) aufweist, wobei die Generatoreinrichtung (9) einen ersten Generator (10) zur Erzeugung von Stoßwellen und einen zweiten Generator (11) zur Erzeugung von Schall aufweist und die Schallquelle (2; 18, 19) über Schaltmittel (12; 20, 21) an die beiden Generatoren (10, 11) zur Erzeugung unterschiedlicher Arten von Schallwellen anschaltbar ist, **dadurch gekennzeichnet,** daß der zweite Generator (11) zur Erzeugung von Dauerschall, der zur Behandlung des Lebewesens dient, ausgebildet ist.

2. Schallerzeuger nach Anspruch 1, **dadurch gekennzeichnet,** daß die Schallquelle (2; 18, 19) wahlweise an einen der Generatoren (10, 11) anschaltbar ist.

3. Schallerzeuger nach Anspruch 1, **dadurch gekennzeichnet,** daß die Schallquelle aus wenigstens zwei unabhängig voneinander antreibbaren Schallwandlern (18, 19) besteht, von denen jeder an jeweils einen Generator (10, 11) anschaltbar ist.

4. Schallerzeuger nach Anspruch 3, **dadurch gekennzeichnet,** daß, mehrere Schallwandler (18, 19) mittels der jeweiligen Generatoren (10, 11) gleichzeitig antreibbar sind.

5. Schallerzeuger nach einem der Ansprüche 1 bis 4, **dadurch gekennzeichnet,** daß der zweite Generator (11) zur Erzeugung von intermittierendem Dauerschall betreibbar ist.

6. Schallerzeuger nach einem der Ansprüche 1 bis 5, **dadurch gekennzeichnet,** daß die Schallquelle durch wenigstens einen piezoelektrischen Wandler (2; 18, 19) gebildet ist.

7. Schallerzeuger nach Anspruch 6, **dadurch gekennzeichnet,** daß zur Erzeugung von Stoßwellen ein Hochspannungsimpulse abgebender Generator (10) und zur Erzeugung von Dauerschall bzw. intermittierendem Dauerschall ein eine periodische bzw. intermittierende periodische Wechselspannung liefernder Generator (11, 14) vorgesehen ist.

## Claims

1. Sound generator for the treatment of a living being with focused sound waves, having a sound source (2; 18, 19) which can be operated electrically, focusing means (5) for the sound waves issuing from the sound source (2; 18, 19) and an electrical generator device (9) operating the sound source (2; 18, 19), with the generator device (9) having a first generator (10) for generating impulse waves and a second generator (11) for generating sound and the sound source (2; 18, 19) being capable of being connected by way of switching means (12; 20, 21) to the two generators (10, 11) for generating different types of sound waves, characterized in that the second generator (11) is constructed for the generation of continuous sound which serves the treatment of the living being.

2. Sound generator according to claim 1, characterized in that the sound source (2; 18, 19) can be selectively connected to one of the generators (10, 11).

3. Sound generator according to claim 1, characterized in that the sound source consists of at least two sound transducers (18, 19) which can be operated independently of one another, each of which can be connected to a respective generator (10, 11).

4. Sound generator according to claim 3, characterized in that several sound transducers (18, 19) can be operated simultaneously by means of the respective generators (10, 11).

5. Sound generator according to one of claims 1 to 4, characterized in that the second generator (11) can be operated for the generation of intermittent continuous sound.

6. Sound generator according to one of claims 1 to 5, characterized in that the sound source is formed by at least one piezoelectrical transducer (2; 18, 19).

7. Sound generator according to claim 6, characterized in that to generate impulse waves a generator (10) emitting high-voltage pulses is provided and to generate continuous sound and intermittent continuous sound a generator (11, 14) supplying a periodical or intermittent periodical alternating voltage is provided.

## Revendications

1. Générateur acoustique pour le traitement d'un être vivant avec des ondes acoustiques focalisées, qui comporte une source acoustique (2;18,19) devant être activée électriquement, des moyens de focalisation (5) pour les ondes acoustiques émises par la source acoustique (2;18,19), et un dispositif générateur électrique (9) alimentant la source acoustique (2;18,19), et dans lequel le dispositif générateur (9) contient un premier générateur (10) servant à produire des ondes de choc, et un second générateur (11) servant à produire un son, et la source acoustique (2;18,19) peut être raccordée par l'intermédiaire de moyens de commutation (12; 20,21) aux deux générateurs (10,11) pour produire différents types d'ondes acoustiques, caractérisé par le fait que le second générateur (11) est agencé de manière à produire un son permanent, qui est utilisé pour le traitement de l'être vivant.

2. Générateur acoustique suivant la revendication 1, caractérisé par le fait que la source acoustique (2;18, 19) peut être raccordée au choix à l'un des deux générateurs (10,11).

3. Générateur acoustique suivant la revendication 1, caractérisé par le fait que la source acoustique est constituée par au moins deux transducteurs acoustiques (18,19) pouvant être activés indépendamment l'un de l'autre et dont chacun peut être raccordé à un générateur (10,11).

4. Générateur acoustique suivant la revendication 3, caractérisé par le fait que plusieurs transducteurs acoustiques (18,19) peuvent être activés simultanément au moyen du générateur respectif (10,11).

5. Générateur acoustique suivant la revendication 1 à 4, caractérisé par le fait que le second générateur (11) peut être activé de manière à produire un son continu intermittent.

6. Générateur acoustique suivant l'une des revendications 1 à 5, caractérisé par le fait que la source acoustique est formée par au moins un transducteur piézoélectrique (2;18,19).

7. Générateur acoustique suivant la revendication 6, caractérisé par le fait que pour la production d'ondes de choc, il est prévu un générateur (10) délivrant des impulsions à haute tension, et pour la production d'un son continu ou d'un son continu intermittent, il est prévu un générateur (11,14) délivrant une tension alternative périodique ou intermittente et périodique.
